**Europäisches Patentamt**

**European Patent Office**

**Office européen des brevets**

(19)

(11) Numéro de publication: **0 173 631**
**B1**

(12) **FASCICULE DE BREVET EUROPÉEN**

(45) Date de publication du fascicule du brevet:
18.05.88

(21) Numéro de dépôt: 85420127.4

(22) Date de dépôt: 12.07.85

(51) Int. Cl.⁴: **B 01 D 57/02,** G 01 N 27/26,
**A 61 M 1/34**

(54) Appareil de fractionnement par électrophorèse de solutions contenant des protéines.

(30) Priorité: 06.08.84 FR 8412579

(43) Date de publication de la demande:
05.03.86 Bulletin 86/10

(45) Mention de la délivrance du brevet:
18.05.88 Bulletin 88/20

(84) Etats contractants désignés:
BE CH DE GB IT LI NL SE

(56) Documents cité:
EP-A-0 052 391
FR-A-2 131 859
GB-A-505 752
GB-A-505 753
US-A-2 801 962

(73) Titulaire: RHONE- POULENC RECHERCHES, 25
Quai Paul Doumer, F-92408 Courbevoie Cedex (FR)

(72) Inventeur: Poulhes, Marie- Andrée, Cantoinet,
F-12420 Sainte- Genevieve (FR)
Inventeur: Lafaille, Jean- Pierre, 9, rue des Alisiers,
F-31650 Saint- Orens (FR)
Inventeur: Pourrat, Jacques, 7, boulevard
Montplaisir, F-31400 Toulouse (FR)
Inventeur: Sanchez, Victor, 1, rue Léonard de
Vinci, F-31520 Toulouse (FR)

(74) Mandataire: Vogt, Bernard, RHONE- POULENC
INTERSERVICES Service Brevets Chimie Centre
de Recherches de Saint- Fons B.P. 62, F-69192
Saint- Fons Cédex (FR)

LIBER, STOCKHOLM 1988

EP 0 173 631 B1

**0 173 631**

## Description

La présente invention concerne un dispositif ou appareil de fractionnement d'une solution coprerant des protéines en vue de séparer au moins deux groupes de substances dissoutes dans celle-ci; elle vise un dispositif de fractionnement par électrophorèse, utilisant un champ électrique.

On à déjà utilisé en laboratoire l'électrophorèse pour fractionner une solution et obtenir des fractions liquides, enrichies en substances contenues dans la solution initiale; l'intérêt essentiel de ce type de procédé est qu'il conserve les propriétés spécifiques des substances fractionnées sans les dénaturer et également qu'il conduit à de faibles consommations énergétiques. On pourra en particulier se reporter au brevet FR publié sous le n° 2 131 859, au brevet US n° 2 801 962, au brevet US n° 2 878 178, au brevet US n° 3 989 613 ou encore au brevet GB 936 805 pour prendre connaissance de plusieurs modes de réalisation de dispositifs permettant la mise en oeuvre de tels procédés d'électrophorèse.

Toutefois, ces dispositifs ne permettent d'obtenir que des rendements relativement médiocres, les durées de fonctionnment nécessaires pour obtenir un taux de fractionnement donné étant souvent très longues.

Ainsi, en pratique, les dispositifs connus cités ci-avant ne permettent pas de profiter pleinement des avantages que fournissent les procédés d'électrophorèse (faible consommation énergétique, absence de dénaturation des substances fractionnées) en raison des défauts inhérents à chacun de ces dispositifs, qui s'opposent à une mise en oeuvre industrielle. De plus, pour la plupart des dispositifs de fractionnement connus, il est nécessaire d'effectuer une préparation préalable de la solution initiale qui doit être diluée et dialysée, en général pour ajuster son pH, en vue d'autoriser un fractionnement efficace. Ces opérations de dialyse sont longues et délicates et sources de contaminations bactériennes des solutions.

Une amélioration sensible de ces dispositifs de fractionnement par électrophoràse à été apportée par l'appareil décrit dans le brevet américain 4 437 967. Cependant il s'est avéré que cet appareil, de par la conception de ses chambres de fractionnement ($C_1$, $C_2$ ...) obtenues chacune à partir de deux éléments (15 et 16), était de réalisation compliquée et ne permettait pas des fractionnements conduisant par exemple à une fraction enrichie contenant plus de 74 % de gamma-globulines par rapport à la teneur en cet élément du sérum bovin introduit dans l'appareil, si ce n'est avec des debits d'alimentation inférieurs ou égaux à 95 ml/h.

Le but de la présente invention est donc un appareil ou dispositif présentant tous les avantages de l'appareil selon le brevet américain 4 437 967 précité, étant plus simple dans sa réalisation et permettant des fractionnements plus poussés à surfaces de membrane égales; il est ainsi possible de recueillir une fraction contenant, dans les conditions de surface de l'exemple 1 du brevet américain 4 437 967, 80 % des gamma-globulines introduites, cette fraction ne contenant par ailleurs pas plus de 10 % de l'albumine introduite, tandis que l'autre fraction contient 90 % de l'albumine introduite et seulement 20 % des gamma-globulines introduites, lors d'une opération de fractionnement de plasma humain ou de sérum bovin, avec un débit d'alimentation de l'ordre de 156 ml/h, soit 1,6 fois plus grand.

Un autre but de la présente invention est un appareil très bien adapté pour le fractionnement du plasma humain, notammet lorsqu'on veut traiter le plasma d'une personne présentant par exemple des anomalies au niveau de ses immuno-globulines, par exemple des ses gamma-globulines, que l'on veut éliminer.

Un autre but de la présente invention est un dispositif ou appareil avec lequel il est possible de traiter en continu un patient dont le sang présente des anomalies, par exemple au niveau des ses immuno-globulines et plus spécialement de ses béta ou gamma-globulines que l'on cherche à éliminer.

A titre d'exemples d'anomalies sanguines qui peuvent être traitées sur le plasma d'un patient avec le dispositif selon la présente invention on peut citer les maladies dans lesquelles on prélève le plasma d'un malade pour le remplacer par le plasma d'une autre personne bien portante. Ce procédé est désigné de façon plus générale par le terme consacré dans la pratique par "échange plasmatique".

Un autre avantage de l'appareil selon la présente invention est qu'il permet de traiter en continu le plasma d'un malade sans avoir à lui réinjecter un autre plasma. Il suffit avec l'appareil selon la présente invention de réinjecter au patient une solution de substitution de composition ionique adéquate dont le volume et la composition ionique correspondent sensiblement à ceux de la fraction éliminée (tandis qu'on réinjecte au malade l'autre fraction, car ses protéines ne sont pas dénaturées).

Un autre avantage de l'appareil selon la présente invention est de permettre de traiter le plasma d'un patient de façon rapide.

Un autre avantage de l'appareil selon la présente invention provient du fait, que dans son adaptation spécifique du traitement du plasma d'un malade, son volume "mort" (c'est-à-dire le volume total pour remplir les compartiments de fractionnement) est faible, inférieur à 400 cm$^3$, de preference aux environs de 200 cm$^3$.

Un autre avantage de l'appareil selon la présente invention provient également du fait que dans l'application ci-avant mentionnée du traitement du plasma humain, on peut se passer des compartiments dits de garde repérés par $G_1$ et $G_2$ (page 6, ligne 10) de la demande de brevet européen 52 391 (correspondant au brevet américain 4 437 967 déjà cité) situés entre les chambres de fractionnement extrêmes et les compartiments d'électrode.

Il a maintenant été trouvé et c'est ce qui fait l'objet de la présente invention un appareil de fractionnement par électrophorèse, utilisable plus spécialement pour le fractionnement de solutions de protéines, notamment celles du plasma humain, en deux fractions liquides, du type comprenant:
- deux plaques d'extrémité (1, 2),
- deux électrodes (5, 6),

2

- deux cadres (3, 4) définissant des compartiments d'électrodes, situés entre les deux plaques d'extrémité (1, 2), chaque cadre étant adjacent à une plaque d'extrémité et à une membrane (10) et comortant des moyens (8, 9) pour l'introduction et l'évacuation dans sa partie centrale (7) creuse d'une solution ionique qui vient au contact de l'électrode,

- des moyens de contention permettant de maintenir serrés entre aux de manière étanche les plaques (1, 2), cadres (3, 4) et membranes (10),

ledit appareil étant caractérisé en ce qu'il comprend:

- au moins une plaque (11) de fractionnement située entre les deux membranes (10) des compartiments d'électrodes, ladite plaque (11) de faible épaisseur comportant au moins une fente (14) étroite traversant ladite plaque et définissant entre les deux membranes (10) une chambre de fractionnement de la solution de protéines à traiter, cette fente (14), en position verticale ou inclinée dans un appareil en fonctionnement, étant reliée à des moyens (17) d'introduction de la solution de protéines à fractionner, lesdits moyens d'introduction débouchant dans ladite fente (14) à au moins 1 cm de sa partie basse (15) et à au plus la moitié de sa longueur L mesurée à partir de cette partie basse (15), ladite fente (14) étant reliée vers son extrémité supérieure (16) à des moyens d'évacuation de la fraction du fluide introduit se dirigeant vers le haut et étant reliée vers son extrémité inférieure (15) à des moyens d'évacuation de la fraction de fluide introduit se dirigeant vers le bas.

La description de l'appareil selon la présente invention sera mieux comprise à l'aide des figures ci-après, qui illustrent de façon schématique et sans échelle déterminée un mode de réalisation d'un tel appareil.

D'autre part les exemples joints illustrent différents modes d'utilisation de cet appareil, plus spécialement pour le fractionnement du plasma humain.

La figure 1 est une vue d'ensemble d'un appareil selon la présente invention.

La figure 2 représente un mode de réalisation d'une plaque de fractionnement.

La figure 3 est une coupe brisée selon 3-3 de la figure 2.

La figure 4 représente les positions relatives d'une plaque de fractionnement et d'un cadre d'électrode.

La figure 5 représente partiellement une variante de réalisation d'une plaque de fractionnement.

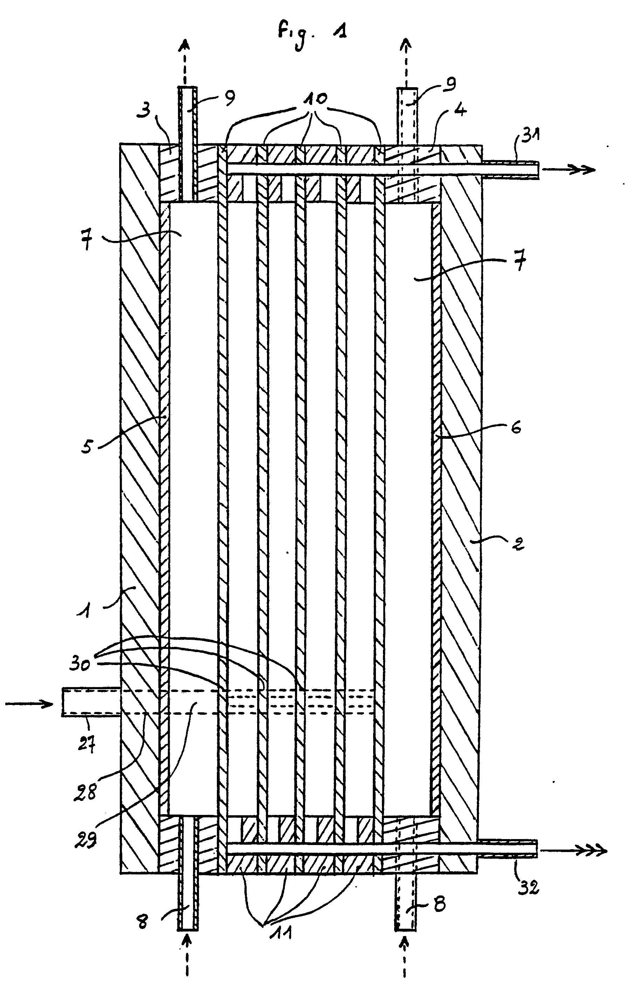

L'appareil ou dispositif selon la présente invention dont un mode de realisation est représente figures 1 à 4 comprend deux plaques d'extrémité (1 et 2), chacune d'elles étant adjacente à un cadre (3, 4) et à une électrode (5, 6) reliée électriquement à un générateur de courant continu non représenté. Chaque cadre (3, 4) comprend une partie centrale creuse (7) et des tubulures (8 et 9) d'introduction et d'évacuation d'une solution ionique devant circuler au contact de l'électrode (5, 6). Chaque cadre (3, 4) est adjacent, par sa face opposée à celle au contact de la plaque d'extrémité (1 ou 2), à une membrane (10) et définit un compartiment dit d'électrode à l'intérieur duquel circule la solution ionique; ce compartiment d'électrode, qui peut être désigné par le chiffre de référence (7) correspordant à la partie creuse du cadre, est ainsi délimitée par la paroi interne de la plaque d'extrémité (1 ou 2) et par la membrane (10). Entre ces deux cadres (3 et 4), situés entre les plaques d'extrémités (1, 2), on trouve des plaques (11) dites de fractionnement, dont le nombre est de quatre pour l'appareil représenté figure 1. Bien entendu cet appareil selon la présente invention peut comporter moins ou plus de plaques (11). Comme on peut le voir sur la figure 1 chaque plaque (11) est adjacente, par chacune de ses deux faces principales (12, 13), à une membrane (10).

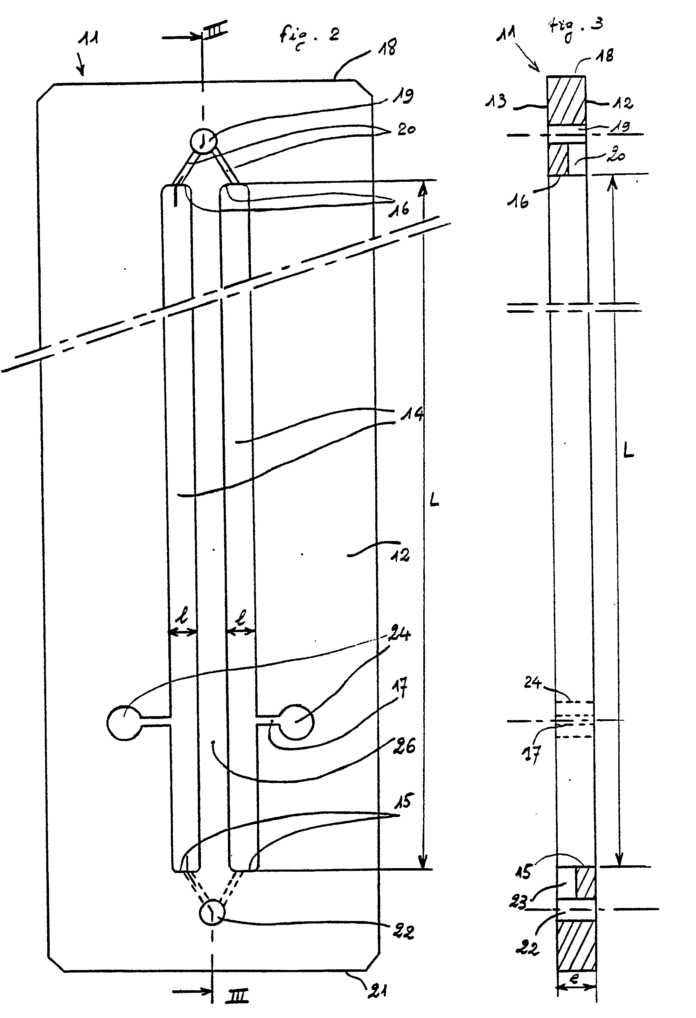

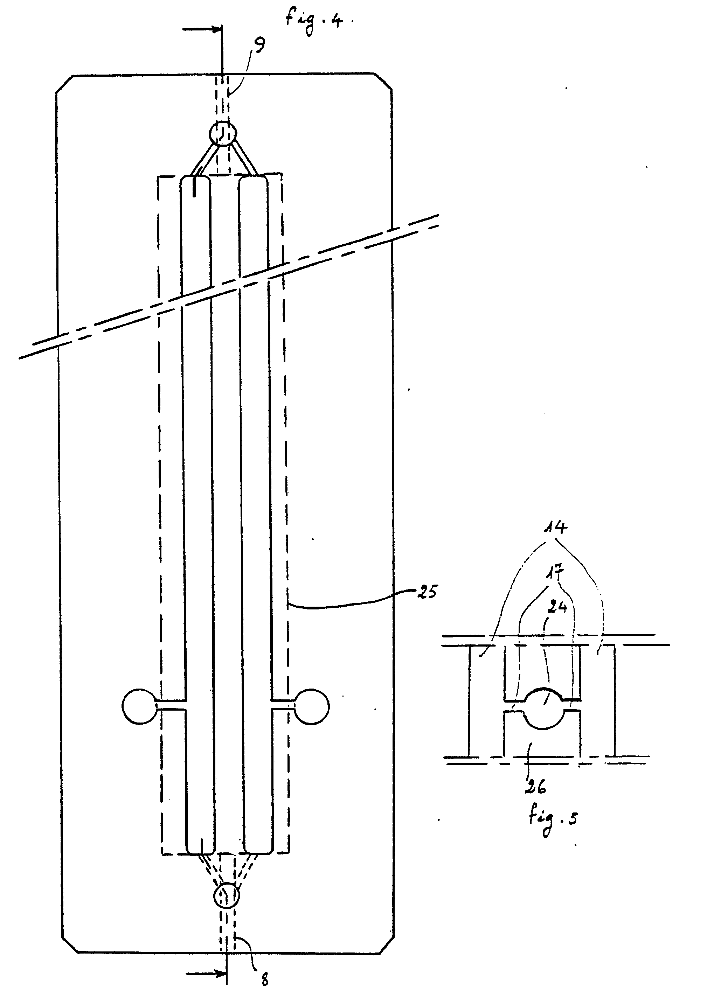

Chaque plaque (11), telle que représentée figures 2 et 3, comprend deux fentes (14) traversant ladite plaque et définissant entre les deux membranes (10) deux chambres de fractionnement de la solution de protéines à séparer. Chaque fente (14) est étroite, ce qui signifie que sa largeur 1 est petite par rapport à sa longueur L, le rapport 1/L étant inférieur à 1/5, de préférence inférieur à 1/15. Chaque chambre de fractionnement est ainsi de volume longiforme.

Dans un appareil en fonctionnement l'axe longitudinal de chaque fente (14) est disposé verticalement par rapport au sol ou est incliné (de même que chaque face 12, 13 de la plaque 11) d'un certain angle par rapport au sol, ce qui signifie que chaque fente, sur sa longueur L, a une partie basse (15) correspondant à son extrémité inférieure par rapport au sol, et une partie haute (16) correspondant à son extrémité supérieure par rapport au sol. Chaque fente est reliée à des moyens (17) d'introduction, dans cette dernière, de la solution de protéines à fractionner, lesdits moyens (17) débouchant dans ladite fente à un endroit situé à au moins 1 cm de sa partie basse (15) et à au plus la moitié de sa longueur L mesurée à partir de la partie basse (15) de la fente (14). De préférence les moyens (17) d'introduction de la solution à fractionner débouchant dans chaque fente (14) à un endroit situé à au moins 2 cm au-dessus de sa partie basse (15) et à au plus au quart de la longueur L de la fente. Les moyens (17) d'introduction sont donc plus proches de la partie basse (15) de chaque fente que de sa partie haute (16).

Les moyens (17) d'intrcduction du fluide à fractionner à l'intérieur de chaque fente (14) comprennent par exemple, tel que cela est représenté, un passage (17) étroit traversant la plaque, ledit passage (17) étant relié à une ouverture (24) traversant aussi la plaque. De préférence le passage (17) est plus étroit que la largeur de la fente (14) et que le diamètre de l'ouverture (24) avantageusement circulaire. Bien que dans le mode de réalisation de la plaque (11) de fractionnement représentée figures 2 et 3 il y ait deux ouvertures (24), il est bien sur possible de ne prévoir qu'une ouverture (24) située entre les deux fentes (14), cette ouverture (24) se trouvant alors sur la languette (26) qu'elle traverse, cette languette (26) étant entre les deux fentes (14). Dans ce cas l'ouverture (24) située sur la languette (26) est reliée à chaque fente (14) par deux passages (17) diamètralement opposés sur la circonférence de cette ouverture (24), chaque passage (17) débouchant dans une fente (14). Dans ce mode de réalisation, représenté partiellement figure 5, il est avantageux que le cadre (3) comprenne une languette correspondant à celle (26) de chaque plaque (11) de fractionnement et qu'un

3

passage (29) [dont il sera question plus loin] en alignement avec l'ouverture (24), soit prévu dans cette languette du cadre (3).

Chaque plaque (11) de fractionnement comprend de plus:
- vers son extrémité supérieure (18) des moyens d'évacuation de la fraction de la solution introduite en (17) se dirigeant vers le haut de la plaque, ces moyens comprenant par exemple un trou (19) traversant la plaque et des canaux (20) ou rainures reliant chaque fente (14) à ce trou,
- vers sen extrémité inférieure (21) des moyens d'évacuation de la fraction de la solution introduite en (17) et se dirigeant vers le bas de la plaque, ces moyens comprenant par exemple un trou (22) traversant la plaque (11) et des canaux (23) ou rainures reliant chaque fente (14) à ce trou (22).

Il est avantageux, pour un meilleur fractionnement de la solution contenant des protéines introduite dans l'appareil, que les moyens d'évacuation (23) de la fraction se dirigeant vers le has de chaque fente (14) soient du côté de migration sous l'influence du champ électrique des protéines chargées, c'est-à-dire vers la face de chaque plaque (11) la plus proche de l'électrode jouant le rôle d'anode, tandis que les moyens d'évacuation (20) de la fraction se dirigeant vers le haut de chaque fente (14) sont plus proches ou sur l'autre face de chaque plaque. Avec l'appareil représenté figure 1, l'électrode (5) est ainsi avantageusement l'anode, lors du fractionnement du plasma humain.

Dans l'appareil tel que décrit ci-avant les membranes (10) n'ont des orifices de passage qu'à leurs endroits en correspondance avec les trous (19 et 22) et aux endroits correspondant aux ouvertures (24) traversant les plaques (11) et en communication avec les moyens (17) d'introduction de la solution à fractionner dans les fentes (14).

Pour des raisons d'étanchéité, les deux plaques (11) de fractionnement adjacents aux cadres (3, 4) de l'appareil décrit ci-avant ont leurs canaux (20 et 23) sur la même face de chaque plaque. Ainsi la plaque (11) adjacente au cadre (3) à ses canaux (20 et 23) qui sont tous deux sur la face de la plaque non adjacente au cadre considéré, tandis que la plaque (11) adjacente au cadre (4) à ses canaux (20, 23) qui sont tous deux sur la face de la plaque non adjacente au cadre considéré. Pour des raisons d'étanchéité également, les deux plaques (11) adjacentes aux cadres (3, 4) ont des passages (17) qui ne traversent pas les plaques et de ce fait les plaques (11) ont leur face [proches des cadres (3, 4)] planes et non traversées par les passages (17) qui ainsi n'occupent qu'une partie de l'épaisseur de ces plaques. Ces détails n'ont pas été représentés figure 1 pour des raisons de simplification.

En ce qui concerne les cadres (3 et 4), comme cela ressort de la figure 1 et de la figure 4 dans laquelle le pourtour de la partie centrale creuse (7) est représentée par la ligne en pointillés (25) [le cadre (3) étant supposé derrière une plaque (11) de fractionnement] il est suffisant que la partie centrale creuse (7), dont la dimension correspond sensiblement à celle de l'électrode (5 ou 6) soit égale ou un peu plus large que la distance maximale entre la partie la plus eloignée des fentes (14).

L'appareil tel que précédemment décrit comprend des moyens de contention (non représentés) permettant de maintenir serrés entre eux les différents cadres, membranes et plaques définis ci-avant. Ces moyens de contention peuvent être par exemple un système de serrage à vis, entre deux plateaux, tel que celui représenté en perspective à la figure 1 du brevet américain 4 437 967 déjà cité. De façon avantageuse le dispositif de contention peut consister en des plaques latérales serrant les cadres (3, 4), les membranes (10) et les plaques (11) de fractionnement entre les plaques (1 et 2) d'extrémité. De telles plaques latérales sont représentées par exemple dans les brevets français 2 114 731 (à la figure 8), 2 482 055 (à la figure 8, repère 28) et européen 23 188.

Pour la mise en oeuvre de l'appareil précédemment décrit on opère de la façon suivante en se rapportant plus particulièrement à la figure 1 qui est une vue d'ensemble en coupe suivant la ligne brisée III-III de la plaque de la figure 2:
- la solution ionique est mise en circulation dans chaque compartiment (7) d'électrode, par des moyens non représentés, la solution entrant dans chaque chambre par la tubulure (8) et en ressortant par la tubulure (9),
- chaque électrode (5 et 6) est mise à la tension électrique souhaitée,
- la solution de protéines à fractionner est introduite en continu dans l'appareil par les deux tubulures (27) dont une seule est représentée figure 1. Chaque tubulure (27) est en alignement avec les ouvertures (24) des plaques (11) de fractionnement et la solution de protéines va jusqu'à ces ouvertures (24) des plaques (11) de fractionnement après avoir traversé des ouvertures correspondantes (28) dans la plaque d'extrémité (1), - (29) dans le cadre (3) et - (30) dans les membranes (10). La solution de protéines à fractionner passe alors dans les passages (17) des plaques (11) de fractionnement et arrive alors simultanément dans les fentes (14), c'est-à-dire dans les chambres de fractionnement comprises entre deux membranes (10) successives.

La solution de protéines se divise alors en continu en deux fractions:
- une fraction ascendante, enrichie en protéines d'un certain type, cette fraction montant vers la partie haute (16) de chaque fente (14), passant par les canaux (20) dans les trous (19) et ensuite allant jusqu'à la tubulure (31) après avoir traversé les membranes (10) par leurs trous ajdacents aux trous (19),
- une fraction descendante, appauvrie en protéines du type dont la fraction ascendante s'est enrichie, cette fraction allant vers la partie tasse (15) de chaque fente (14), passant par les canaux (23) dans les trous (22) et sortant de l'appareil par la tubulure (32), après avoir traversé les membranes (10) par leurs trous en aligmement avec les trous (22).

Plus généralement la fraction descendante s'enrichit des protéines chargées sous l'influence du champ électrique.

Sur la figure 1 la solution de protéines à fractionner entrant en continu dans l'appareil est schématisée par la flèche pleine à un 5 chevron, la fraction ascendante sortant en continu de l'appareil est représentée par une flèche pleine à deux chevrons, tandis que la fraction descendante sortant de l'appareil en continu est schématisée par une flèche pleine à trois chevrons.

L'appareil selon la présente invention peut faire l'objet de nombreuses variantes. Il est par exemple possible qu'une plaque d'extrémité et le cadre adjacent ne forment en réalité qu'une seule pièce. Les plaques (11) de fractionnement peuvent comporter plus ou moins que deux fentes (14); à titre d'exemple chaque plaque (11) peut avoir quatre fentes (14).

A titre de variante de réalisation chaque plaque (11) de fractionnement, au lieu d'avoir des canaux (20) sur sa face (12) et des canaux (23) sur sa face (13) opposée, peut comprendre des canalisations internes à la plaque (11) considérée, lesdites canalisations reliant chaque extrémité (15 ou 16) de chaque fente (14) aux trous (19 ou 22). De préférence, pour un meilleur fractionnement, lesdites canalisations internes ci-avant, qui sont dans l'épaisseur e de chaque plaque (11) sont plus proches, vers l'extrémité inférieure (15) de chaque fente, de la face de chaque plaque la plus proche de l'électrode jouant le rôle d'anode. Les canalisations internes proches de l'extrémité (16) supérieure de chaque fente (14) sont proches de la face de la plaque opposée à celle la plus proche de l'anode. D'autre part les passages (17) traversant les plaques (11) et reliant les ouvertures (24) aux fentes (14) peuvent être à l'intérieur de chaque plaque (11). Par ses deux variantes les deux plaques (11) de fractionnement adjacentes aux cadres (3 et 4) peuvent être identiques aux autres plaques (11) de l'appareil.

En ce qui concerne les membranes utilisables dans l'appareil selon la présente invention, il s'est avéré que les membranes semi-perméables telles que celles utilisées dans les appareils d'osmose inverse étaient très convenables. De telles membranes sont largement commercialisées. A titre indicatif on peut citer les membranes semi-perméables vendues sous la marque "CELLOPHANE", qui sont en cellulose régénérée. Des membranes telles que celles précédemment définies et ayant de très faibles quantités de groupements anioniques, par exemple des groupements sulfoniques $SO_3^-$, sont également convenables.

L'appareil selon la présente invention peut avoir des dimensions de plaques de fractionnement (11) très variables en fonction de l'application envisagée. Cet appareil peut bien sûr être utilisé pour le fractionnement industriel de solutions de protéines. Il se prête particulièrement bien au fractionnement du plasma humain. Avec un appareil selon la présente invention il a été ainsi possible d'éliminer en deux heures, 50 % et même 70 % des gamma-globulines du plasma de malades atteints de myélome IgG (en opérant en continu sur les malades).

Les plaques (11) peuvent être en tout matériau diélectrique neutre vis-à-vis des solutions initiales à traiter, par exemple en polyméthacrylate de méthyle ou "PLEXIGLAS".

Généralement le champ électrique entre deux membranes (10) successives est entre 250 et 400 V/m.

Généralement la largeur 1 des fentes (14) est comprise entre 0,5 et 2 cm, de préférence entre 0,75 et 1,25 cm pour des membranes (10) non renforcées par exemple par un tissu interne.

Pour le fractionnement du plasma humain en continu avec des membranes en CELLOPHANE, il s'est avéré que l'épaisseur des plaques (11) de fractionnement était avantageusement comprise entre 1,3 et 1,7 mm.

Pour un débit d'entrée du plasma humain à fractionner de l'ordre de 25 à 40 ml/mn, il a été trouvé qu'il fallait fournir une différence de potentiel entre électrodes de 30 à 40 Volts, l'intensité du courant traversant les chambres étant de 1,8 à 2 Ampères.

En ce qui concerne les électrodes elles ne doivent pas introduire des ions contaminants dans la solution à fractionner. A titre d'exemples elles peuvent être en platine (sous forme de grilles) ou en carbone ou graphite (sous forme de plaques).

## Exemple 1:

On à procédé au fractionnement de plasma humain disposé dans un récipient en faisant arriver ce plasma dans un appareil tel que précédemment décrit figures 1 à 4, comprenant 15 plaques (11) selon la figure 2, c'est-à-dire avec deux fentes (14) dont chaque fente avait une largeur 1 de 1 cm, une longueur L de 24 cm et l'épaisseur de la plaque était de 0,15 cm. Chaque fente était disposée verticalement par rapport au sol.

Le volume mort de l'appareil était de 108 ml.

Les électrodes (5, 6) étaient des grilles de platine, l'électrode (5) proche des tubulures (27) était l'anode.

Les membranes (10) étaient de marque CELLOPHANE, référence 600 N.

L'épaisseur des cadres (3, 4) était de 1 cm.

La différence de potentiel (d.d.p.) entre électrodes était de 30 Volts.

L'intensité du courant traversant les chambres était de 1,6 Ampère.

La hauteur des passages (17) de chaque plaque était au 1/6 de la longueur L des fentes (14), c'est-à-dire à 4 cm de la partie basse (15) de chaque fente (14).

La solution ionique circulant dans les compartiments (7) d'électrodes était une solution de NaCl à 9/1 000 et son débit était de 3,5 litres à l'heure (l/h).

La solution de plasma humain circulait dans l'appareil à un débit d'entrée (en 27) de 900 ml/h. Le débit de la fraction sortant de la tubulure (31) c'est-à-dire de la fraction ascendante ou fraction haute était de 450 ml/h ; ce débit sera appelé dH.

Le débit de la fraction sortant de la tubulure (32), c'est-à-dire de la fraction descendante ou fraction basse était aussi de 450 ml/h; ce débit sera appelé dB.

Comme on peut le voir:

$$R = \frac{dH}{dB} = 1$$

Les deux fractions recueillies en (31) et en (32) étaient chacune recueillie dans un récipient indépendant et les dosages ont été faites sur chaque fraction obtenue.

Dans les conditions opératoires ci-dessus définies et avec l'appareil décrit on a obtenu les résultats suivants (dans un intervalle de temps quelconque):

$$(I) = \frac{\text{quantité de gamma-globulines récupérées (en 31)}}{\text{quantité de gamma-globulines introduite en (27)}} \times 100 = 55\ \%$$

$$(II) = \frac{\text{quantité d'albumine récupérée (en 32)}}{\text{quantité d'albumine introduite en (27)}} \times 100 = 92\ \%$$

Ainsi dans la fraction haute il n'y a pas plus de 8 % de l'albumine introduite dans l'appareil, tandis que dans la fraction basse il n'y a pas plus de 45 % des gamma-globulines introduites dans l'appareil.

**Exemple 2:**

On a opéré dans les mêmes conditions que celles de l'exemple 1 sauf en ce qui concerne le débit d'entrée du plasma dans l'appareil qui était de 300 ml/h et en ce qui concerne R qui était égal à 3.

Ainsi dH = 225 ml/h et dB = 75 ml/h.

Les résultats obtenus furent:

I = 80 %
II = 92 %

I et II ayant les mêmes significations qu'à l'exemple 1.

**Exemple 3:**

On a opéré dans les mêmes conditions que celles de l'exemple 2, mais avec des plaques d'épaisseur e de 0,2 cm. Le volume mort de l'appareil était alors de 144 ml.

Les résultats furent:

I = 80 %
II = 90 %

Cet appareil est moins interessant lorsqu'on est branché sur un malade que l'appareil selon l'exemple 2 qui n'a que 108 ml de volume mort.

**Exemple 4:**

On a opéré dans les conditions de l'exemple 1 avec le même appareil sauf en ce qui concerne la hauteur d'introduction du fluide à fractionner dans les fentes (14).

Un essai a été fait avec introduction au point 1/8 L, 1/3 L et 1/2 L, c'est-à-dire à 3 cm, à 8 cm et à 12 cm de la partie basse (15) de chaque fente (14).

Les résultats obtenus apparaissent ci-après:

| Introduction | 1/8 L | 1/3 L | 1/2 L |
|---|---|---|---|
| I | 55 % | 55 % | 55 % |
| II | 93 % | 80 % | 75 % |

**0 173 631**

**Exemple 5:**

Un essai a été fait en continu, pendant deux heures, sur un malade en état comateux en début de séance, atteint de myélome IgG. Le plasma de ce malade, en début de séance, comprenait 29 g/l de gamma-globulines et 32 g/l d'albumine.

L'appareil utilisé était le même que celui de l'exemple 1, sauf en ce qui concerne le nombre de plaques (11) de fractionnement qui avait été amené à 30.

Les conditions opératoires étaient les mêmes que celles de l'exemple 1 (R était égal à 1) sauf en ce qui concerne le fait que le plasma était retiré en continu à partir du sang du malade et que le plasma entrait dans l'appareil en (27) avec un débit de 30 ml/m (millilitre par minute).

Ainsi on soutirait en continu le sang du malade, dans un circuit extracorporel, on faisait passer son sang sur une séparateur à membrane, de type connu, pour lui extraire une partie de son plasma, qui traversait la membrane (la récupération de ce plasma se faisait à un débit de 30 ml/m), tandis que la fraction du sang ne traversant pas la membrane était retournée en continu au malade par une ligne dite de retour du sang (cette dernière fraction contenant les éléments figurés du sang et la partie du plasma qui n'avait pas traversé la membrane).

Ainsi le plasma du malade traversant en continu la membrane de l'appareil séparateur était introduit en continu dans l'appareil selon la présente invention par les tubulures (27).

La fraction du plasma du malade recueillie à la sortie haute (31) de l'appareil était récupérée dans un récipient, tandis que la fraction sortant par la tubulure basse (32) était renvoyée en continu au malade par la ligne de retour du sang, tout en ajoutant en continu dans cette ligne de retour de la fraction sortant par la tubulure (32) une solution de substitution (soit 1 800 ml en deux heures, puisque R = 1) préparée en mélangeant:

- 1 500 ml de NaCl à 0,15 mol/litre
- 750 ml d'eau
- 60 ml de Na H CO$_3$ à 0,1 mol/litre
- 9 ml de KCl à 10 % en poids
- 15 ml de gluconate de calcium à 10 % en poids

Le malade s'est réveillé en cours de séance.

En fin de séance la quantité de gamma-globulines récupérées par la tubulure (31) haute de l'appareil représentait 55 % de la quatité de gamma-globulines présentes dans le plasma du malade en début de séance. La quantité d'albumine récupérée dans cette même fraction (sortant en haut de l'appareil) représentait 20 % de l'albumine présente dans le plasma du malade en début de séance.

**Revendications**

1. Appareil de fractionnement par électrophorèse, utilisable plus spécialement pour le fractionnement de solutions contenant des protéines en deux fractions liquides, du type comprenant:
- deux plaques d'extrémité (1, 2),
- deux électrodes (5, 6),
- deux cadres (3, 4) définissant des compartiments d'électrodes, situés entre les deux plaques d'extrémité (1, 2), chaque cadre étant adjacent à une plaque d'extrémité et à une membrane (10) et comportant des moyens (8, 9) pour l'introduction et l'évacuation dans sa partie centrale (7) creuse d'une solution ionique qui vient au contact de l'électrode,
- des moyens de contention permettant de maintenir serrés entre eux de manière étanche les plaques (1, 2), cadres (3, 4) et membranes (10), ledit appareil étant caractérisé en ce qu'il comprend en outre au moins une plaque (11) de fractionnement située entre les deux membranes (10) des compartiments d'électrodes, ladite plaque (11) de faible épaisseur comportant au moins une fente (14) étroite (c'est-à-dire ayant une largeur l petite par rapport à sa longeur L) traversant ladite plaque et définissant entre les deux membranes (10) une chambre de fractionnement de la solution à traiter, cette fente (14), en position verticale ou incliné e dans un appareil en fonctionnement, étant reliée à des moyens (17) d'introduction de la solution de protéines à fractionner, lesdits moyens d'introduction débouchant dans ladite fente (14) à au moins 1 cm de sa partie basse (15) et à au plus la moitié de sa longueur L mesurée à partir de cette partie basse (15), ladite fente (14) étant reliée vers son extrémité supérieure (16) à des moyens (20) d'évacuation de la fraction du fluide introduit se dirigeant vers le haut et étant reliée vers son extrémité inférieure (15) à des moyens (23) d'évacuation de la fraction de fluide introduit se dirigeant vers le bas.

2. Appareil selon la revendication 1, caractérisé en ce que les moyens (17) d'introduction de la solution à fractionner débouchent dans chaque fente (14) à au moins 2 cm de sa partie basse (15) et à au plus le quart de sa longueur L mesurée à partir de la partie basse (15).

3. Appareil selon l'une quelconque des revendications précédentes, caractérisé en ce qu'il comprend une pluralité de plaques (11) de fractionnement, chaque plaque (11) de fractionnement étant séparée de la plaque

7

adjacente par une membrane (10).

4. Appareil selon l'une quelconque des revendications précédentes, caractérisé en ce que les moyens (17) d'introduction de la solution à fractionner comprennent pour chaque plaque (11) au moins une ouverture (24) traversant la plaque considérée, ladite ouverture (24) étant en communication par un passage (17) avec au moins une fente (14) de la plaque (11), chaque membrane (10) ayant un trou correspondant à chaque ouverture (24) d'une plaque (11).

5. Appareil selon l'une quelconque des revendications précédentes, caractérisé en ce que les moyens (23) d'évacuation de la fraction du fluide se dirigeant vers le bas (15) de chaque fente (14) sont plus proches de la face de chaque plaque (11) proche de l'électrode jouant le rôle d'anode, tandis que les moyens (20) d'évacuation de la fraction se dirigeant vers le haut (16) de chaque fente (14) sont vers l'autre face de chaque plaque.

6. Appareil selon l'une quelconque des revendications précédentes, caractérisé en ce que les moyens d'évacuation de la fraction se dirigeant vers la partie basse (15) de chaque fente (14) de chaque plaque (11) comprennent au moins un canal ou canalisation (23) en communication avec la partie basse (15) de la fente (14) et avec un trou (22) traversant chaque plaque (11), en ce que les moyens d'évacuation de la fraction se dirigeant vers la partie haute (16) de chaque fente (14) de chaque plaque (11) comprennent au moins un canal ou canalisation (20) en communication avec la partie haute (16) de la fente (14) et avec un trou (19) traversant chaque plaque (11), les membranes (10) adjacentes aux plaques (11) ayant des trous en correspondance avec les trous (19) et (22).

7. Appareil selon l'une quelconque des revendications précédentes, caractérisé en ce que l'épaisseur e de chaque plaque (11) est comprise entre 1,3 et 1,7 m.

8. Appareil selon l'une quelconque des revendications précédentes, caractérisé en ce que le rapport l/L est inférieur à 1/5, l représentant la largeur d'une fente (14).

9. Utilisation de l'appareil selon l'une quelconque des revenidications précédentes pour le fractionnement en continu du plasma humain.


**Patentansprüche**

1. Vorrichtung zur Fraktionierung durch Elektrophorese, insbesondere verwendbar zur Fraktionierung von Lösungen, enthaltend Proteine in zwei flüssigen Fraktionen, von der Art aufweisend:
- zwei Endplatten (1, 2),
- zwei Elektroden (5, 6),
- zwei Einfassungen (3, 4), die zwischen den beiden Endplatten (1, 2) liegende Elektrodenräume begrenzen, wobei jede Einfassung benachbart einer Endplatte und einer Membran (10) ist und Mittel (8, 9) zur Einführung und zur Abführung einer Ionenlösung in ihren hohlen zentralen Teil (7) umfaßt, die in Kontakt mit der Elektrode tritt,
- wobei Zusammenhaltemittel es gestatten, die Platten (1, 2), Einfassungen (3, 4) und Membranen (10) auf dichte weise zwischeneinander zusammengedrückt zu halten,
- wobei die Vorrichung dadurch gekennzeichnet ist, daß sie außerdem aufweist
- wenigstens eine zwischen den beiden Membranen (10) der Elektrodenräume liegende Fraktionierungsplatte (11), wobei die Platte (11) mit geringer Dicke wenigstens einen schmalen Schlitz (14) umfaßt, der eine in bezug auf seine Länge L kleine Breite l besitzt, die Platte durchquert und zwischen den beiden Membranen (10) eine Kammer zur Fraktionierung der zu behandelnden Lösung begrenzt, wobei dieser Schlitz (14) in vertikaler oder geneigter Position in einer Vorrichtung in Betrieb mit Mitteln (17) zur Einführung der Lösung zu fraktionierender Proteine verbunden ist, wobei die Mittel zur Einführung in den Schlitz (14) wenigstens 1 cm von seinem unteren Teil (15) und bei höchstens der Hälfte von dessen Länge L, gemessen von diesem unteren Teil (15) her, münden, wobei der Schlitz (14) in Richtung seines oberen Endes (16) mit Mitteln (20) zum Abführen der Fraktion des sich nach oben richtenden, eingeführten Fluids verbunden ist und in Richtung seines unteren Endes (15) mit Mitteln (23) zum Abführen der Fraktion von sich nach unten richtendem, eingeführten Fluid verbunden ist.

2. Vorrichtung nach Anspruch 1, dadurch gekennzeichnet, daß die Mittel (17) zum Einführen der zu fraktionierenden Lösung in jeden Schlitz (14) bei wenigstens 2 cm von dessen unterem Teil (15) und bei höchstens dem Viertel von dessen Länge L, gemessen von dessen unterem Teil (15) aus, einmünden.

3. Vorrichtung nach einem beliebigen der vorhergehenden Ansprüche, dadurch gekennzeichnet, daß sie eine Anzahl von Platten (11) zur Fraktionierung aufweist, wobei jede Platte (11) zur Fraktionierung von der benachbarten Platte durch eine Membran (10) getrennt ist.

4. Vorrichtung nach einem beliebigen der vorhergehenden Ansprüche, dadurch gekennzeichnet, daß die Mittel (17) zum Einführen der zu fraktionierenden Lösung für jede Platte (11) wenigstens eine die betrachtete Platte durchquerende Öffnung (24) aufweisen, wobei sich die Öffnung (24) mittels einer Durchführung (17) mit wenigstens einem Schlitz (14) der Platte (11) in Verbindung befindet, wobei jede Membran (10) ein Loch entsprechend jeder Öffnung (24) einer Platte (11) besitzt.

5. Vorrichtung nach einem beliebigen der vorhergehenden Ansprüche, dadurch gekennzeichnet, daß die Mittel (23) zum Abführen der Fraktion des abwärts (15) eines jeden Schlitzes (14) richtenden Fluids näher der Seite einer jeden Platte (11) nahe der die Rolle einer Anode spielenden Elektrode sind, während die Mittel (20) zum Abführen der sich aufwärts (16) eines jeden Schlitzes (14) richtenden Fraktion in Richtung zur anderen Seite einer jeden Platte sind.

6. Vorrichtung nach einem beliebigen der vorhergehenden Ansprüche, dadurch gekennzeichnet, daß die Mittel zum Abführen der sich zum unteren Teil (15) eines jeden Schlitzes (14) einer jeden Platte (11) richtenden Fraktion wenigstens einen Kanal oder Kanalsystem (23) in Verbindung mit dem unteren Teil (15) des Schlitzes (14) und mit einem eine jede Platte (11) durchquerenden Loch (22) aufweisen, dass die Mittel zum Abführen der sich zum oberen Teil (16) eines jeden Schlitzes (14) einer jeden Platte (11) richtenden Fraktion wenigstens einen Kanal oder Kanalsystem (20) in Verbindung mit dem oberen Teil (16) des Schlitzes (14) und mit einem eine jede Platte (11) durchquerenden Loch (19) aufweisen, wobei die den Platten (11) benachbarten Membranen (10) Löcher entsprechend den Löchern (19 und 22) besitzen.

7. Vorrichtung nach einem beliebigen der vorhergehenden Ansprüche, dadurch gekennzeichnet, daß die Dicke e einer jeden Platte (11) einschließlich zwischen 1,3 und 1,7 mm ist.

8. Vorrichtung nach einem beliebigen der vorhergehenden Ansprüche, dadurch gekennzeichnet, daß das Verhältnis l/L kleiner als 1/5 ist, wobei l die Breite eines Schlitzes (14) darstellt.

9. Verwendung der Vorrichtung nach einem beliebigen der vorhergehenden Ansprüche zur ununterbrochenen Fraktionierung des menschlichen Plasmas.

**Claims**

1. Apparatus for fractionation by electrophoresis, which can be used more especially for fractionating protein solutions into two liquid fractions, of the type comprising:
- two end plates (1, 2),
- two electrodes (5, 6),
- two frames (3, 4) which define electrode compartments, situated between the two end plates (1, 2), each frame being adjacent to an end plate and a membrane (10) and incorporating means (8, 9) for the introduction and removal, in its hollow central part (7), of an ionic solution which comes into contact with the electrode,
- holding means which enable the plates (1, 2), frames (3, 4) and membranes (10) to be maintained clamped together in a leakproof manner,
the said apparatus being characterized in that it comprises, in addition
- at least one fractionation plate (11) situated between the two membranes (10) of the electrode compartments, said plate (11) of little thickness incorporating at least one narrow slit (14) (i.e. having a width w which is small compared to its length L) passing through the said plate and defining between the two membranes (10) a chamber for fractionating the solution to be treated, this slit (14), vertical or sloping in position in an apparatus when in operation, being connected to means (17) for introduction of the protein solution to be fractionated, the said means for introduction opening into the said slit (14) at least 1 cm from its bottom (15) and at most at half its length L measured from this bottom (15) the said slit (14) being connected towards, its upper end (16) to means (20) for removing the fraction of the fluid introduced which is directed upwards, and being connected together its lower end (15) to means (23) for removing the fraction of fluid introduced which is directed downwards.

2. Apparatus according to claim 1, characterized in that the means (17) for introducing the solution to be fractionated open into each slit (14) at a point at least 2 cm from its bottom (15) and at most at one-quarter of its length L measured from the bottom (15).

3. Apparatus according to either one of the preceding claims, characterized in that it contains a plurality of fractionation plates (11), each fractionation plate being separated from the adjacent plate by a membrane (10).

4. Apparatus according to any ore of the preceding claims, characterized in that the means (17) for introducing the solution to be fractionated comprise, for each plate (11), at least one aperture (24) passing through the plate in question, the said aperture (24) being in communication through a passage (17) with at least one slit (14) of the plate (11), each membrane (10) having a hole corresponding to each aperture (24) in a plate (11).

5. Apparatus according to any of the preceding claims, characterized in that the means (23) for removal of the fluid fraction which is conveyed towards the bottom (15) of each slit (14) are closer to the face of each-plate (11) which is close to the electrode performing the role of anode, while the means (20) for removal of the fraction which is conveyed towards the top (16) of each slit (14) are towards the other face of each plate.

6. Apparatus according to any one of the preceding claims, characterized in that the means for removing the fraction which is conveyed towards the bottom (15) of each slit (14) of each plate (11) comprise at least one channel or duct (23) in communication with the bottom (15) of the slit (14) and with a hole (22) passing through each plate (11), and in that the means for removal of the fraction which is conveyed towards the top (16) of each slit (14) of each platt (11) comprise at least one channel or duct (20) in communication with the top (16) of the slit (14) and with a hole (19) passing through each plate (11), the membranes (10)

9

adjacent to the plates (11) having holes corresponding with the holes (19) and (22).

7. Apparatus according to any one of the preceding claims, characterized in that the thickness t of each plate (11) is between 1.3 and 1.7 mm.

8. Apparatus according to any one of the preceding claims, characterized in that the ratio w/L is less than 1 : 5, w denoting the width of a slit (14).

9. Use of the apparatus according to any one of the preceding claims for the continuous fractionation of human plasma.

Fig. 1

fig. 2

fig. 3

fig. 4

fig. 5